# EUROPEAN PATENT APPLICATION

(11) **EP 1 254 661 A1**
(43) Date of publication of application: **06.11.2002**
(21) Application number: 02008620.3
(22) Date of filing: 17.04.2002
(51) Int. Cl.: A61K 31/445, A61P 25/14, A61P 25/16

(54) **Method for preventing dyskinesias with selective NMDA-Antagonists**

(30) Priority: 23.04.2001 US 285870 P
(71) Applicant: WARNER-LAMBERT COMPANY, Morris Plains New Jersey 07950 (US)
(72) Inventor: Meltzer, Leonard Theodore, Ann Arbor, MI 48105 (US); Bedard, Paul Joseph, Quebec City, G1R 5H5, Quebec (CA)
(74) Representative: Tesch, Rudolf, Dr.

(57) **Abstract**

Dyskinesias in humans are prevented by administering a therapeutically effective dose of a NR1A/2B site-selective NMDA receptor antagonist compound to a human.

## Description

### BACKGROUND OF THE INVENTION

### Field of the Invention

This invention relates to a method for preventing disease-related or drug-induced dyskinesias.

### Related Background Art

Parkinson's disease (PD) is one of the most common neurodegenerative diseases, affecting almost 1% of the population over 65 (Tanner C.M., Aston D.A., Epidemiology of Parkinson's disease and akinetic syndromes. *Curr*. *Opin. Neural*., 2000;13:427-430). Recently, a major step in its treatment was achieved as illustrated by the results of three clinical trials which showed that the early use of dopamine agonists conclusively delayed the occurrence of dyskinesias and motor complications (Parkinson Study Group, Pramipexole vs levodopa as initial treatment far Parkinson's disease: A randomized controlled trial. *JAMA*, 2000;284:1931-1938; Rascol O., Brooks D.J., Korczyn A.D., De Deyn P.P., Clarke C.E., Lang A.E., A five-year study of the incidence of dyskinesia in patients with early Parkinson's disease who were treated with ropinirole or levodopa. *N. Engl. J. Med*., 2000;342:1484-1491; Rinne U.K., Bracco F., Chouza C., Dupont E., Gershanik O., Marti Masso J.F., et al., Early treatment of Parkinson's disease with cabergoline delays the onset of motor complications. Results of a double-blind levodopa controlled trial. *Drugs*, 1998;55(Suppl 1): 23-30). Furthermore, these studies showed that dyskinesias were further reduced in patients who were not supplemented with L-Dopa but were under dopamine agonist monotherapy until the end of the follow-up period (3 to 5 years). However, following Frucht S., Rogers J.D., Greene P.E., Gordon M.F., Fahn S., Falling asleep at the wheel: motor vehicle mishaps in persons taking pramipexole and ropinirole. *Neurology*, 1999;52:1908-1910 published observation about episodes of sudden onset of sleep (sleep attacks) in PD patients on pramipexole and ropinirole, the early hope to delay dyskinesias using dopamine agonists is at least tempered. In addition, given the fact that with disease progression the possibility of increasing dopamine agonist dosage will be compromised because of such side-effects, L-dopa is therefore often introduced to maximize clinical benefits and may ultimately lead to the emergence of dyskinesias. In fact, in a large proportion of patients, L-Dopa therapy is plagued by the development of dyskinesias which are relatively common, with a prevalence ranging between 30% to 80% after long-term L-Dopa administration (Blanchet P.J., Allard P., Gregoire L., Tardif F., Bedard P.J., Risk factors for peak dose dyskinesia in 100 levodopa-treated parkinsonian patients. *Can. J. Neurol*. *Sci*., 1996:23:189-193; Fahn S. The spectrum of levodopa-induced dyskinesias. *Ann*. *Neurol.,* 2000;47(Suppl 1):S2-92000; Grandas F., Galiano M.L., Tabernero C., Risk factors for levodopa-induced dyskinesias in Parkinson's disease. *J. Neurol.,* 1999;246:1127-1133). Dyskinesias can become more disabling than parkinsonism itself and still represent a major shortcoming of pharmacological management of PD. It is somewhat embarrassing to have to agree with a recent review by Ferreira J.J., Rascol O., Prevention and therapeutic strategies for levodopa-induced dyskinesias in Parkinson's disease. *Curr. Opin*. *Neurol*., 2000;13:431-436 that, in spite of tremendous efforts by different research teams all over the world, we still have no satisfactory treatment for dyskinesias in clinical practice. Hence, there is a pressing need to find a preventive strategy to avoid the appearance of motor fluctuations and dyskinesias.

The mechanisms underlying levodopa-induced dyskinesias are not completely understood. Several lines of evidence have recently indicated that alteration of the N-methyl-D-aspartate (NMDA) receptor function as a consequence of chronic intermittent L-Dopa therapy may play an important role. Indeed, dopamine depletion and subsequent L-Dopa treatment induce adaptive responses in the striatum involving striatal glutamatergic function. In L-Dopa-treated parkinsonian patients, analysis of postmortem tissue showed an increased binding to striatal NMDA but not to kainate or AMPA receptors (Ulas J., Weihmuller F.B., Brunner L.C., Joyce J.N., Marshall J.F., Cotman C.W., Selective increase of NMDA-sensitive glutamate binding in the striatum of Parkinson's disease, Alzheimer's disease, and mixed Parkinson's disease/Alzheimer's disease patients: an autoradiographic study. *J. Neurosci.,* 1994;14:6317-624). Furthermore, an up-regulation of striatal NR2B subunit was found in dyskinetic MPTP primates (Di Paolo T., Calon F., Morissette M., Ghribi O., Grondin R., Goulet G., et al., Striatal glutamate receptors in dyskinetic MPTP monkeys. *Soc. Neuorosci.*, 2000; New Orleans), as revealed by quantitative autoradiography using [³H]-Ro256981, a potent and selective antagonist of NMDA receptors containing NR2B subunits (Mutel V., Buchy D., Klingelschmidt A., Messer J., Bleuel Z., Kemp J.A., Richards J.G., In vitro binding properties in rat brain of [³H]Ro 25-6981, a potent and selective antagonist of NMDA receptors containing NR2B subunits. *J. Neurochem.*, 1998;70:2147-255). In a rodent model of PD, (the 6-hydroxydopamine(6-OHDA)-lesioned rats), repeated L-Dopa treatment induces an enhanced motor response to challenge with dopamine-like agents and this has been suggested to represent a rodent counterpart of levodopa-induced dyskinesias (Henry B., Crossman A.R, Brotchie J.M., Characterization of enhanced behavioral responses to L-DOPA following repeated administration in the 6-hydroxydopamine-lesioned rat model of Parkinson's disease. *Exp. Neurol*., 1998;151(2)(Jun):334-342). In this rat model, receptor binding studies have also demonstrated a significant increase in NR2B subunit expression after long-term L-Dopa administration (Ravenscroft P., Brotchie J., Alterations in striatal NR1 and NR2B NMDA receptor subunit expression in the 6-OHDA-lesioned rat model of L-DOPA-induced dyskinesia. *Parkinsonism & Related Disorders*, 1999;5(Suppl):S42). Furthermore, enhanced tyrosine phosphorylation of NR2B-containing NMDA receptors following L-Dopa administration in medium spiny output neurons, has been reported (Menegoz M., Lau L.F., Herve D., Huganir R.L., Girault J.A., Tyrosine phosphorylation of NMDA receptor in rat striatum: effects of 6-OH-dopamine lesions. *Neuroreport*, 1995;7:125-128; Oh J.D., Russell D.S., Vaughan C.L., Chase T.N., Russell D., Enhanced tyrosine phosphorylation of striatal NMDA receptor subunits: effect of dopaminergic denervation and L-DOPA administration. *Brain Res*., 1998;813:150-159; Dunah A.W., Wang Y., Yasuda R.P., Kameyama K., Huganir R.L., Wolfe B.B., Standaert D.G., Alterations in subunit expression, composition, and phosphorylation of striatal N-methyl-D-aspartate glutamate receptors in a rat 6-hydroxydopamine model of Parkinson's disease. *Mol. Pharmacol.*, 2000;57:342-352). Since protein phosphorylation serves as an important regulatory mechanism for NMDA receptors (Suen P.C., Wu K., Xu J.L., Lin S.Y., Levine E.S., Black I.B., NMDA receptor subunits in the postsynaptic density of rat brain: expression and phosphorylation by endogenous protein kinases. *Brain Res. Mol. Brain Res.*, 1998;59:215-228), their synaptic efficacy is increased (Chase T.N., The significance of continuous dopaminergic stimulation in the treatment of Parkinson's disease. *Drugs*, 1998;55(Suppl 1):1-9; Chase T.N., Oh J.D., Striatal dopamine- and glutamate-mediated dysregulation in experimental parkinsonism. *Trends Neurosci.*, 2000;10(Suppl):S86-91). Other NMDA receptor subunits, such as NR1 or NR2A are also altered by denervation and subsequent L-Dopa treatment, but the increase in NR2B mRNA and protein level and/or in its tyrosine phosphorylation only in animals with dyskinesias suggest au causal link. Therefore, there is a probable relationship between the induction of persistant dyskinesias and up-regulation of NMDA receptor containing NR2B subunit. Other factors, including changes in the distribution and/or coupling function of NMDA receptors may contribute to these functional changes, which may ultimately lead to the development of motor complications (Dunah, supra., 2000; Chase and Oh, supra., 2000).

In line with these observations, behavioral studies using NMDA receptor antagonists in experimental parkinsonism and in PD patients suggest that blockade of NMDA receptor can reduce L-Dopa-induced dyskinesias. In PD patients, NMDA receptor antagonists such as dextromethorphan or amantadine reduced L-Dopa-induced dyskinesias (Verhagen M.L., Blanchet P.J., van den Munckhof P., Del Dotto P., Natte R., Chase T.N., A trial of dextromethorphan in parkinsonian patients with motor response complications. *Mov. Disord*., 1998a;13:414-417; Verhagen M.L., Del Dotto P., Natte R., van den Munckhof P., Chase T.N., Dextromethorphan improves levodopa-induced dyskinesias in Parkinson's disease. *Neurology*, 1998b;51:203-206; Verhagen Metman L., Del Dotto P., van den Munckhof P., Fang J., Mouradian M.M., Chase T.N., Amantadine as treatment for dyskinesias and motor fluctuations in Parkinson's disease. *Neurology*, 1998c;50:1323-1326; Blanchet P.J., Konitsiotis S., Chase T.N., Amantadine reduces levodopa-induced dyskinesias in parkinsonian monkeys. *Mov. Disord*., 1998;13:798-802), and the beneficial effects of amantadine on motor response complications was maintained for at least 1 year after treatment initiation in one study (Metman L.V., Del Dotto P., LePoole K., Konitsiotis S., Fang J., Chase T.N., Amantadine for levodopa-induced dyskinesias: a 1-year follow-up study. *Arch. Neurol.*, 1999;56:1383-1386). In primate models of PD, selective blockade of NMDA receptors containing the NR2B subunit reduced dyskinesias (Papa S.M., Chase T.N., Levodopa-induced dyskinesias improved by a glutamate antagonist in Parkinsonian monkeys. *Ann. Neurol*., 1996;39:574-578) and produced an antiparkinsonian effect (Steece-Collier K., Chambers L.K., Jaw-Tsai S.S., Menniti F.S., Greenamyre J.T., Antiparkinsonian actions of CP-101,606, an antagonist of NR2B subunit-containing N-methyl-d-aspartate receptors. *Exp*. *Neurol*., 2000;163:239-43; Nash J.E., Fox S.H., Henry B., Hill M.P., Peggs D., McGuire S., Maneuf Y., et al., Antiparkinsonian actions of ifenprodil in the MPTP-lesioned marmoset model of Parkinson's disease. *Exp. Neural*., 2000;165:136-142). Furthermore, 6-OHDA-lesioned rats when given long-term intermittent L-Dopa treatment gradually begin to manifest response alterations to dopamine-like agents (shortening in the duration of the response to dopaminergic challenge and other changes) reminiscent of the wearing-off and on-off phenomena seen in PD patients (Papa S.M., Engber T.M., Kask A.M., Chase T.N., Motor fluctuations in levodopa treated parkinsonian rats: relation to lesion extent and treatment duration. *Brain Res.*, 1994;662:69-74). Attempts to elucidate the molecular mechanisms by which NMDA receptors may play a role in these motor complications have been carried out in this rodent model by Chase and his team in Bethesta. The authors have found that L-Dopa-induced motor fluctuations could be completely reversed and more interestingly prevented by the systemic administration of MK-801, a nonselective NMDA receptor antagonist (Boldry R.C., Papa S.M., Kask A.M., Chase T.N., MK-801 reverses effects of chronic levodopa on D1 and D2 dopamine agonist-induced rotational behavior. *Brain Res.,* 1995;692(1-2)(Sep 18):259-264; Marin C., Papa S., Engber T.M., Bonastre M., Tolosa E., Chase T.N., MK-801 prevents levodopa-induced motor response alterations in parkinsonian rats. *Brain Res.*, 1996;736:202-205; Papa S.M., Boldry R.C., Engber T.M., Kask A.M., Chase T.N., Reversal of levodopa-induced motor fluctuations in experimental parkinsonism by NMDA receptor blockade. *Brain Res.*, 1995;701:13-18). Both motor fluctuations and dyskinesias seem to share a common pathophysiological mechanism, that is altered or deficient gating of glutamatergic inputs to the striatum by dopamine (Calon F., Hadj T.A., Blanchet P.J., Morissette M., Grondin R., Goulet M., Doucet J.P., et al., Dopamine-receptor stimulation: biobehavioral and biochemical consequences. *Trends Neurosci.*, 2000;23:S92-100; Chase and Oh, supra., 2000). Hence, taken together these findings provide strong clinical/behavioral evidence for a link between NMDA receptor stimulation and long-term L-Dopa side effects. Studies similar to those performed in rodents have yet to be extended to primates.

Monkeys rendered parkinsonian by the neurotoxin 1,2,3,6-tetrahydro-1-methyl-4-phenylpyridine (MPTP) provide an exceptional tool to study dyskinesias, since when given L-Dopa chronically they develop involuntary movements phenotypically identical to dyskinesias observed in PD patients. In the present work using this model, it was investigated whether selective NMDA receptor antagonism could prevent the appearance of dyskinesias. The induction of dyskinesias in eight de novo (drug naive) parkinsonian monkeys divided in two groups was compared. One group received L-Dopa alone and the other one L-Dopa plus CI-1041 (n=4 for each group). CI-1041, a substituted piperidine related to ifenprodil, is >1000-fold selective for NR1A/2B cloned NMDA receptors (Whittemore E.R., Ilyin V.I., Woodward R.M., Electrophysiological characterization of CI-1041 on cloned and native NMDA receptors. *Soc. Neurosci*., 2000; New Orleans). It acts at an allosteric modulation site as a novel NMDA antagonist that blocks NR2B-containing NMDA receptors with a nanomolar affinity (24 nM) (Whittemore, supra., 2000). The antiparkinsonian effects of CI-1041 alone and in combination with L-Dopa were assessed in the MPTP-treated monkeys. CI-1041, at 3 to 30 mg/kg, did not exhibit any antiparkinsonian activity when administered alone and did not produce a consistent alteration in the antiparkinsonian effects of L-Dopa (Christoffersen C.L., Wright J.L., Kesten S.R., Meltzer L.T., Effects of CI-1041, a selective NMDA 1A/2B receptor antagonist, in MPTP treated monkeys. *Soc. Neurosci.,* 2000; New Orleans). However, CI-1041 produced a replicable reduction in L-Dopa-induced dyskinesias of 30% to 50% in 2 of 3 monkeys. In the 6-OHDA-lesioned hemiparkinsonian rat model, CI-1041 potentiated the contraversive rotation induced by L-Dopa and increased both the peak rate and duration of rotation (Serpa K.A., Wright J.L., Kesten S.R., Meltzer L.T., Antiparkinsonian (AP) effects of CI-1041 (PD196860), a subtype selective NMDA antagonist, in the 6-OHDA rat model. *Soc. Neurosci.*, 2000; New Orleans). This effect was maintained following 9 days of repeated co-administration. CI-1041 likewise potentiated rotation induced by the direct acting dopamine agonists, SKF 38393 and quinpirole, suggesting the antiparkinsonian effects are not due to effects on L-Dopa metabolism (Serpa, supra., 2000). When given alone, CI-1041 increased both contraversive and ipsiversive rotations (Serpa, supra., 2000). Applicants demonstrate that co-treatment with CI-1041 prevented dyskinesias, a finding which conclusively points out the contribution of NMDA receptor-mediated mechanisms in L-Dopa-induced dyskinesias genesis and gives further evidence that prevention of this disabling side effect is possible.

PCT International Publication No. WO 96/37226 discloses treatment of Parkinson's disease with a combination of site-selective NMDA receptor antagonist compounds and L-Dopa. Administration of the antagonist compounds allowed use of lower amounts of L-Dopa. However, there is no suggestion that NR1A/2B site-selective NMDA receptor antagonist compounds could be administered to prevent the side effects accompanying normal doses of L-Dopa.

PCT International Publication No. WO/US 98/19357 (incorporated herein by reference) discloses treatment of disease-related or drug-induced dyskinesias with NR1A/2B site-selective NMDA receptor antagonist compounds. However, there is no suggestion that NR1A/2B site-selective NMDA antagonists could be administered to prevent the side effects accompanying normal doses of L-Dopa including L-Dopa-induced dyskinesias.

Methods of prevention of dyskinesias with compounds which are NR1A/2B site-selective NMDA receptor antagonists would be desirable.

### SUMMARY OF THE INVENTION

This invention is directed to a method for preventing dyskinesias, said method comprising administering to a subject, preferably a human, suffering therefrom a therapeutically effective amount of a NR1A/2B site-selective NMDA receptor antagonist compound.

In a preferred embodiment of this invention, the compound is 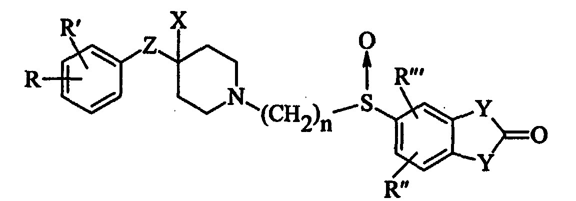 or stereoisomers or a pharmaceutically acceptable salt thereof, wherein R and R' are independently selected from the group consisting of hydrogen, hydroxy, alkyl, halogen, nitro, cyano, carboxaldehyde, aldehyde amine, lower alkoxy carbonylmethyl, hydroxy lower alkyl, aminocarbonylmethyl, hydrazinocarbonylmethyl, acetamido, aryl, aralkyl, amino, a halogenated alkyl group, a lower alkyl amino group, a lower alkyl amino group, or a lower alkoxy group;
- R" and R"': are independently selected from the group consisting of hydrogen, hydroxy, alkyl, halogen, amino, a halogenated alkyl group, a lower alkyl amino group, or a lower alkoxy group;
- X: is hydrogen or hydroxy;
- Z: is -CH₂- or 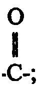
- N: is 2 to 4; and
- Y: is O, NH, or S.

In another preferred embodiment of this invention, the compound is selected from the group consisting of 6-{2-[4-(4-chloro-benzyl)-piperidin-1-yl]-ethanesulfinyl}-3H-benzooxazol-2-one; 6- {2-[4-(4-fluoro-benzyl)-piperidin-1-yl]-ethanesulfinyl}-3H-benzooxazol-2-one; 6-{2-[4-(4-methyl-benzyl)-piperidin-1-yl]-ethanesulfinyl}-3H-benzooxazol-2-one; 6-[2-(4-benzyl-piperidin-1-yl)-ethanesulfinyl]-3H-benzooxazol-2-one; 6-{2-[4-(4-methoxybenzyl)-piperidin-1-yl]-ethanesulfinyl}-3H-benzooxazol-2-one; 6-{2-[4-(3,4-dichlorobenzyl)-piperidin-1-yl]-ethanesulfinyl}-3H-benzooxazol-2-one; 6-{2-[4-(2-fluorobenzyl)-piperidin-1-yl]-ethanesulfinyl}-3H-benzooxazol-2-one; 6-[2-(4-benzyl-4-hydroxypiperidin-1-yl)-ethanesulfinyl]-3H-benzooxazol-2-one; 6-{2-[4-(4-fluorobenzyl)-4-hydroxy-piperidin-1-yl]-ethanesulfinyl}-3H-benzooxazol-2-one; 6-[2-(4-benzoylpiperidin-1-yl)-ethanesulfinyl]-3H-benzooxazol-2-one; 6-{2-[4-(2,3-difluorobenzyl)-piperidin-1-yl]-ethanesulfinyl}-3H-benzooxazol-2-one; 6-{2-[4-(2,4-difluorobenzyl)-piperidin-1-yl]-ethanesulfinyl}-3H-benzooxazol-2-one; 6-{2-[4-(4-trifluoromethylbenzyl)-piperidin-1-yl}-3H-benzooxazol-2-one; 6{2-[4-(2,6-difluorobenzyl)-piperidin-1-yl]-ethanesulfinyl}-3H-benzooxazol-2-one; 6-{2-[4-(2,4-dichlorobenzyl)-piperidin-1-yl]-ethanesulfinyl}-3H-benzooxazol-2-one; N-(4-{1-[2-(2-oxo-2,3-dihydrobenzooxazol-6-sulfinyl)ethyl]piperidin-4-ylmethyl}phenyl)acetamide; 6-[2-(4-benzylpiperidin-1-yl)ethanesulfinyl]-5-chloro-3H-benzooxazol-2-one; 5-chloro-6-{2-[4-(4-fluorobenzyl)piperidin-1-yl]ethanesulfmyl}-3H-benzooxazol-2-one; (+)-6-{2-[4-(4-fluorobenzyl)-piperidine-1-yl]ethanesulfinyl}-3H-benzooxazol-2-one; (-)-6-{2-[4,(4-fluorobenzyl)piperidin-1-yl]ethanesulfinyl}-3H-benzooxazol-2-one and pharmaceutically acceptable salts thereof.

In another preferred embodiment of this invention, the compound is 6-[2-[-(4-fluoro-benzyl)-piperidin-1-yl]-ethanesulfinyl]-3H-benzooxazol-2-one or a pharmaceutically acceptable salt thereof.

In a further preferred embodiment of this invention, the compound is 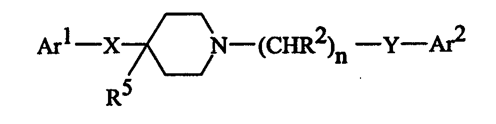 or a pharmaceutically acceptable salt thereof wherein
- Ar¹ and Ar²: are independently aryl or a heteroaryl group, either of which may be independently substituted by one to three of hydroxy, alkyl, halogen, nitro, cyano, carboxaldehyde, aldehyde oxime, lower alkoxy carbonylmethyl, hydroxy lower alkyl, aminocarbonylmethyl, hydrazinocarbonylmethyl, acetamido, aryl, aralkyl, amino, a halogenated alkyl group, a lower alkyl amino group, or a lower alkoxy group;
- X: is -(CHR³)ₘ-, wherein each R³ is independently hydrogen, hydroxy, or a lower alkyl group having 1 to 6 carbon atoms; and m is 0 or 1;
- each R²: is independently hydrogen, hydroxy, or a lower alkyl group having 1 to 6 carbon atoms;
- n: is 1,2,3, or 4;
- Y: is C≡C, O, and SOₚ wherein p is 0, 1, or 2, NR⁴ wherein R⁴ is hydrogen or a lower alkyl group having 1 to 6 carbon atoms, or a single bond; and
- R⁵: is hydrogen or hydroxy.

In another preferred embodiment, the compound has a structure selected from those listed below, their stereoisomers, and their pharmaceutically acceptable salts: 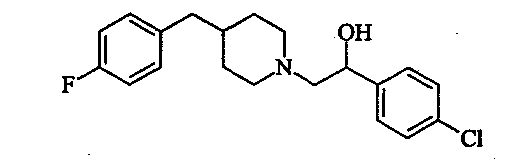 α-(4-chlorophenyl)-4-[(4-fluorophenyl)methyl]-1-piperidineethanol; 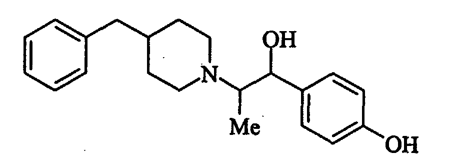 α-(4-hydroxyphenyl)-β-methyl-4-(phenylmethyl)-1-piperidineethanol; 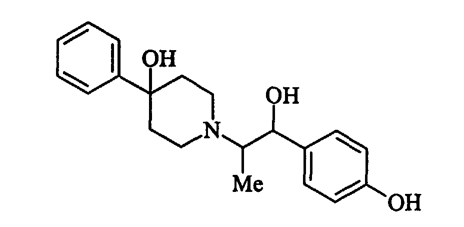 4-hydroxy-α-(4-hydroxyphenyl)-β-methyl-4-phenyl-1-piperidineethanol; 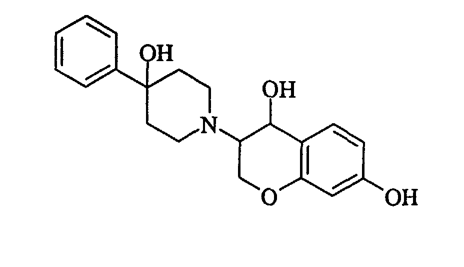 3-[4-(4-phenyl)-4-hydroxypiperidin-1-yl]-chroman-4,7-diol; and 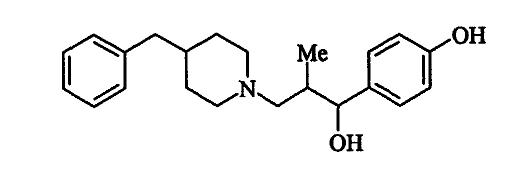 α-(4-hydroxyphenyl)-β-methyl-4-(phenylmethyl)-1-piperidinepropanol.

### BRIEF DESCRIPTION OF THE INVENTION

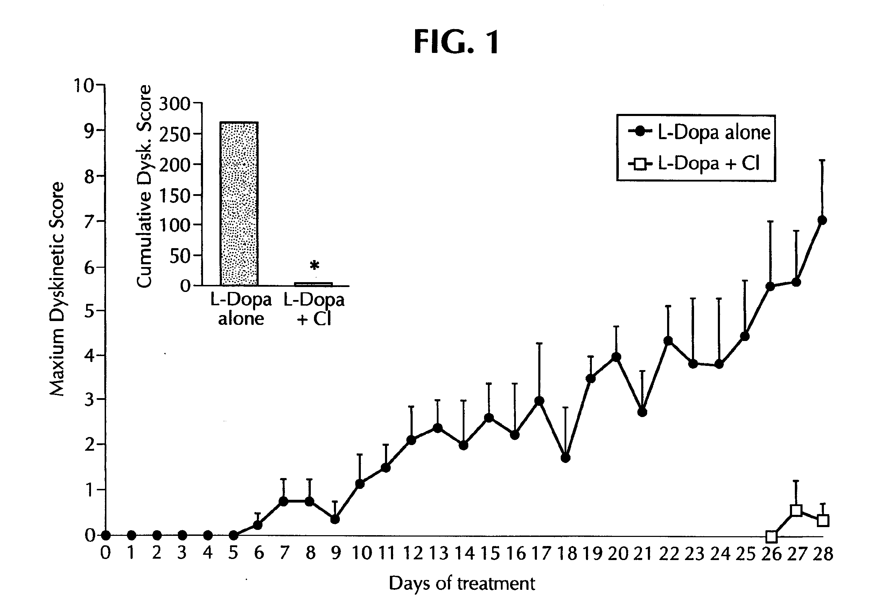

Figure 1 is a graph showing the dyskinetic response alone or with CI-1041 wherein the maximum dyskinetic scores obtained during an observed effect following a given treatment were averaged for all monkeys and the averaged scores were then compared using a nonparametric Mann-Whitney nonpaired test. Scores±SEM, *P<0.018 versus L-Dopa group.

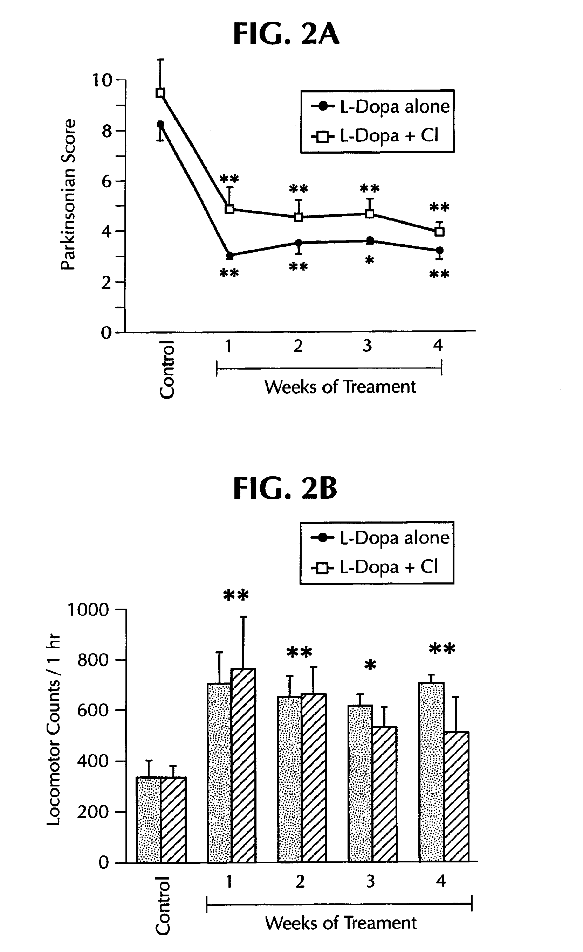

Figure 2 is a graph showing the antiparkinsonian response wherein the minimum parkinsonian scores (A) obtained for each monkey during an observed effect following a given treatment were averaged for all monkeys and compared using a nonparametric Friedman and Mann-Whitney test. Parkinsonian scores: means±SEM; *P<0.05, and **P<0.01 versus control. The total mobility counts (B) recorded individually during 1 hour during the peak effect following a given treatment were averaged for all monkeys. These averaged scores and mobility counts were then compared using an ANOVA for repeated measure followed by a PLSD (least significant difference) test. Counts ± SEM, *P<0.05 and **P<0.01 versus control.

### DETAILED DESCRIPTION OF THE INVENTION

In the method of this invention, dyskinesias are prevented by administration of a therapeutically effective amount of a NR1A/2B site-selective NMDA receptor antagonist compound.

The terms "prevent," "prevention," and "preventing" include preventative (e.g., prophylactic) and palliative treatment or the act of providing preventative or palliative treatment which prevents the development and/or onset of dyskinesias.

The term "subject" means animals, particularly mammals. Preferred subjects are humans.

In a further preferred embodiment of this invention, the compound is 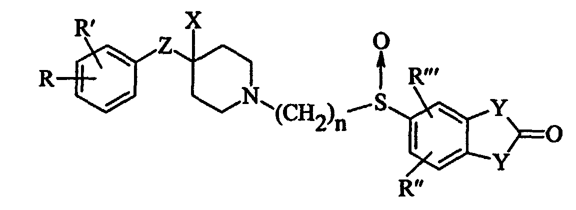 or stereoisomers or a pharmaceutically acceptable salt thereof, wherein R and R' are independently selected from the group consisting of hydrogen, hydroxy, alkyl, halogen, nitro, cyano, carboxaldehyde, aldehyde amine, lower alkoxy carbonylmethyl, hydroxy lower alkyl, aminocarbonylmethyl, hydrazinocarbonylmethyl, acetamido, aryl, aralkyl, amino, a halogenated alkyl group, a lower alkyl amino group, a lower alkyl amino group, or a lower alkoxy group;
- R" and R''': are independently selected from the group consisting of hydrogen, hydroxy, alkyl, halogen, amino, a halogenated alkyl group, a lower alkyl amino group, or a lower alkoxy group;
- X: is hydrogen or hydroxy;
- Z: is -CH₂- or 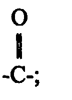
- N: is 2 to 4; and
- Y: is O, NH, or S which are disclosed in WO 00/00197, the disclosures of which are incorporated by reference herein.

In another preferred embodiment of this invention, the compound is selected from the group consisting of 6-{2-[4-(4-chloro-benzyl)-piperidin-1-yl]-ethanesulfinyl}-3H-benzooxazol-2-one; 6-{2-[4-(4-fluoro-benzyl)-piperidin-1-yl]-ethanesulfinyl}-3H-benzooxazol-2-one; 6-{2-[4-(4-methyl-benzyl)-piperidin-1-yl]-ethanesulfinyl}-3H-benzooxazol-2-one; 6-[2-(4-benzyl-piperidin-1-yl)-ethanesulfinyl]-3H-benzooxazol-2-one; 6-{2-[4-(4-methoxybenzyl)-piperidin-1-yl]-ethanesulfinyl}-3H-benzooxazol-2-one; 6-{2-[4-(3,4-dichlorobenzyl)-piperidin-1-yl]-ethanesulfinyl}-3H-benzooxazol-2-one; 6-{2-[4-(2-fluorobenzyl)-piperidin-1-yl]-ethanesulfinyl}-3H-benzooxazol-2-one; 6-[2-(4-benzyl-4-hydroxypiperidin-1-yl)-ethanesulfinyl]-3H-benzooxazol-2-one;6-{2-[4-(4-fluorobenzyl)-4-hydroxy-piperidin-1-yl]-ethanesulfinyl}-3H-benzooxazol-2-one; 6-[2-(4-benzoylpiperidin-1-yl)-ethanesulfinyl]-3H-benzooxazol-2-one; 6-{2-[4-(2,3-difluorobenzyl)-piperidin-1-yl]-ethanesulfinyl}-3H-benzooxazol-2-one; 6-{2-[4-(2,4-difluorobenzyl)-piperidin-1-yl]-ethanesulfinyl}-3H-benzooxazol-2-one; 6-{2-[4-(4-trifluoromethylbenzyl)-piperidin-1-yl}-3H-benzooxazol-2-one; 6{2-[4-(2,6-difluorobenzyl)-piperidin-1-yl]-ethanesulfinyl}-3H-benzooxazol-2-one; 6-{2-[4-(2,4-dichlorobenzyl)-piperidin-1-yl]-ethanesulfinyl}-3H-benzooxazol-2-one; N-(4-{1-[2-(2-oxo-2,3-dihydrobenzooxazol-6-sulfinyl)ethyl]piperidin-4-ylmethyl}phenyl)acetamide; 6-[2-(4-benzylpiperidin-1-yl)ethanesulfinyl]-5-chloro-3H-benzooxazol-2-one; 5-chloro-6-{2-[4-(4-fluorobenzyl)piperidin-1-yl]ethanesulfinyl}-3H-benzooxazol-2-one; (+)-6-{2-[4-(4-fluorobenzyl)-piperidine-1-yl]ethanesulfinyl}-3H-benzooxazol-2-one; (-)-6-{2-[4,(4-fluorobenzyl)piperidin-1-yl]ethanesulfinyl}-3H-benzooxazol-2-one and pharmaceutically acceptable salts thereof.

In another preferred embodiment of this invention, the compound is 6-[2-[-(4-fluoro-benzyl)-piperidin-1-yl]-ethanesulfinyl]-3H-benzooxazol-2-one or a pharmaceutically acceptable salt thereof.

In a further preferred embodiment of this invention, the compound is 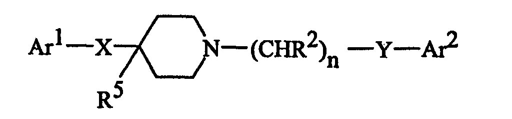 or a pharmaceutically acceptable salt thereof wherein
- Ar¹ and Ar²: are independently aryl or a heteroaryl group, either of which may be independently substituted by one to three of hydroxy, alkyl, halogen, nitro, cyano, carboxaldehyde, aldehyde oxime, lower alkoxy carbonylmethyl, hydroxy lower alkyl, aminocarbonylmethyl, hydrazinocarbonylmethyl, acetamido, aryl, aralkyl, amino, a halogenated alkyl group, a lower alkyl amino group, or a lower alkoxy group;
- X: is -(CHR³)ₘ-, wherein each R³ is independently hydrogen, hydroxy or a lower alkyl group having 1 to 6 carbon atoms; and m is 0 or 1;
- each R²: is independently hydrogen, hydroxy or a lower alkyl group having 1 to 6 carbon atoms;
- n: is 1, 2, 3 or 4;
- Y: is C≡C, O, SOₚ wherein p is 0, 1 or 2, NR⁴ wherein R⁴ is hydrogen or a lower alkyl group having 1 to 6 carbon atoms, or a single bond; and
- R⁵: is hydrogen or hydroxy, which are disclosed in PCT International Publication Nos. WO 97/23216 and WO 97/23214, the disclosures of which are incorporated by reference herein.

In another preferred embodiment of this invention, the compound administered is selected from the compounds having the structures and names listed below, their stereoisomers, and their pharmaceutically acceptable salts: 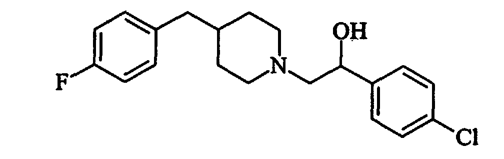 α-(4-chlorophenyl)-4-[(4-fluorophenyl)methyl]-1-piperidineethanol (Eliprodil, available from Synthelabo, Inc, and disclosed in US Patent No. 5,547,963) 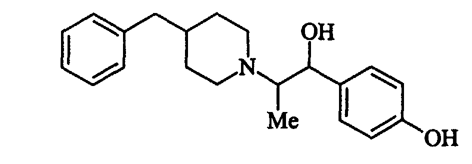 α-(4-hydroxyphenyl)-β-methyl-4-(phenylmethyl)-1-piperidineethanol (Ifenprodil, available from Synthelabo, Inc, and disclosed in US Patent No. 3,509,164) 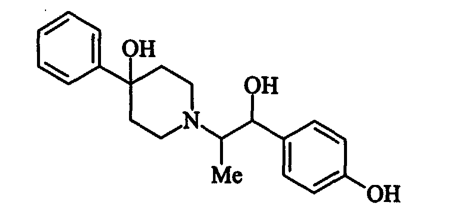 4-hydroxy-α-(4-hydroxyphenyl)-β-methyl-4-phenyl-1-piperidineethanol (optically active form CP-101,606, available from Pfizer, Inc, and disclosed in US Patent No. 5,272,160) 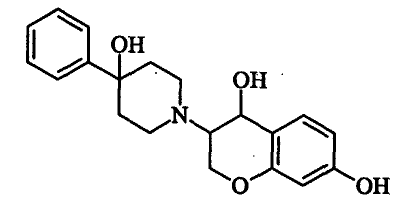 3-[4-(4-phenyl)-4-hydroxypiperidin-1-yl]-chroman-4,7-diol (optically active form CP-283,097, available from Pfizer, Inc) 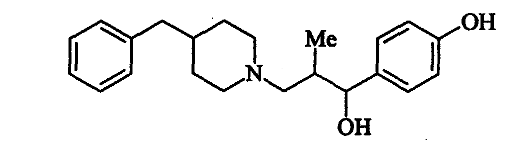 α-(4-hydroxyphenyl)-β-methyl-4-(phenylmethyl)-1-piperidinepropanol (optically active form Ro 25-6981, available from Hoffmann-La Roche, and disclosed in European Patent Application No. EP 648,744).

The most preferred compounds for administration in the method of this invention are 1-[2-(4-hydroxyphenoxy)ethyl]-4-hydroxy-4-(4-methylbenzyl) piperidine hydrochloride, which has the following structure: 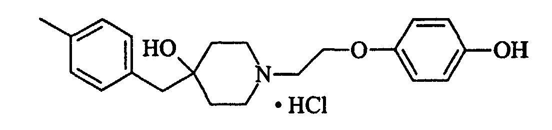 and 1-[2-(4-Hydroxyphenoxy)ethyl]-4-(4-fluorobenzyl)-4-hydroxy-piperidine hydrochloride, which has the following structure: 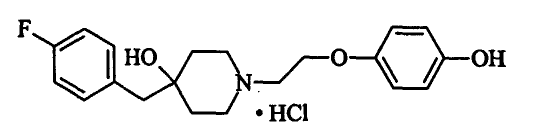 and CP-101,606, [S-(R*,R*)]-4-hydroxy-α-(4-hydroxyphenyl)-β-methyl-4-phenyl-1-piperidineethanol, which has the following structure: 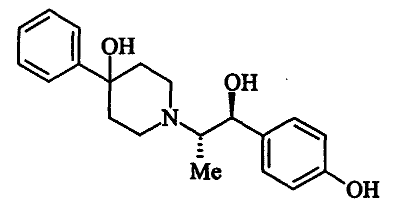 and is described in PCT Application No. WO 96/37226, the disclosure of which is incorporated by reference herein.

The method of the present invention also includes administering a compound or composition as set forth above prior to or concurrent with the administration of L-Dopa, apomorphine, pramipexole, ropinirole, cabergoline, bromocryptine, pergolide, U-91356A, SKF 38393, SKF 82958, A-77636, A-86929, CY-208-243, any other selective or nonselective dopamine or other combined dopamine D1, D2, D3, D4, or D5 receptor agonists, or combinations thereof to prevent the development and/or onset of dyskinesias.

Also included within the scope of the present invention is the use of the non-toxic pharmaceutically acceptable salts of the compounds described herein. Acid addition salts are formed by mixing a solution of the particular compound used in the present invention with a solution of a pharmaceutically acceptable non-toxic acid such as hydrochloric acid, fumaric acid, maleic acid, succinic acid, acetic acid, citric acid, tartaric acid, carbonic acid, phosphoric acid, oxalic acid, and the like.

The pharmaceutical compositions of the present invention may be administered by any means that achieve their intended purpose. For example, administration may be by parenteral, subcutaneous, intravenous, intramuscular, intraperitoneal, transdermal, or buccal routes. Alternatively, or concurrently, administration may be by the oral route. The dosage administered will be dependent upon the age, health, and weight of the recipient, kind of concurrent treatment, if any, frequency of treatment, and the nature of the effect desired.

Suitable dosage levels in the method of this invention for oral administration range from about 100 mg/dose to about 1000 mg/dose. For subcutaneous administration, the suitable level is from about 1 mg/dose to about 200 mg/dose.

The examples which follow are intended as illustrations of certain preferred embodiments of the invention, and no limitation of the invention is implied.

### EXAMPLE 1

### Material and Methods

### Animals and Pretreatments

The experiments were performed in accordance with the standards of the Canadian Council on Animal care using eight female cynomolgus (*Macaca fascicularis*) monkeys weighing 3.0 to 3.5 kg. They were housed separately in individual observation cages equipped with an electronic motility monitoring system (Datascience, St. Paul, Minnesota) in a temperature-controlled room and exposed to a 12-hour light/dark cycle (lights on 6 AM-6 PM). They were fed once daily in the afternoon (certified primate chow and fruits), and water was provided ad libitum. All animals were initially treated with the neurotoxin MPTP (Sigma-Aldrich Canada Ltd, Oakville, Ontario) dissolved in sterile water and injected continuously using Alzet minipumps at a dose of 0.5 mg/24 hours. In general, 2 to 4 weeks are needed to induce sustained parkinsonian features. This method of MPTP delivery is well-tolerated and allows us to reduce both the time to induce parkinsonism and the risk of MPTP manipulation. Recovery was seen in some animals, in this case 2 mg of MPTP was administered once a week, for 1 to 3 weeks. The cumulative dose and the time necessary to achieve this goal were in average 15.6 ± 2.4 mg and 4.2 ± 1 months, respectively. The animals were scored several times a week using a disability scale described below, where the normal state extends from 0 to 2, and maximal disability is 16 points. Treatment was started after the bilateral parkinsonian syndrome had stabilized (i.e., unchanged disability score of 8 or more over a month). This study was undertaken several months after the stable parkinsonian syndrome had developed (for more details on animal characteristics see Table 1).

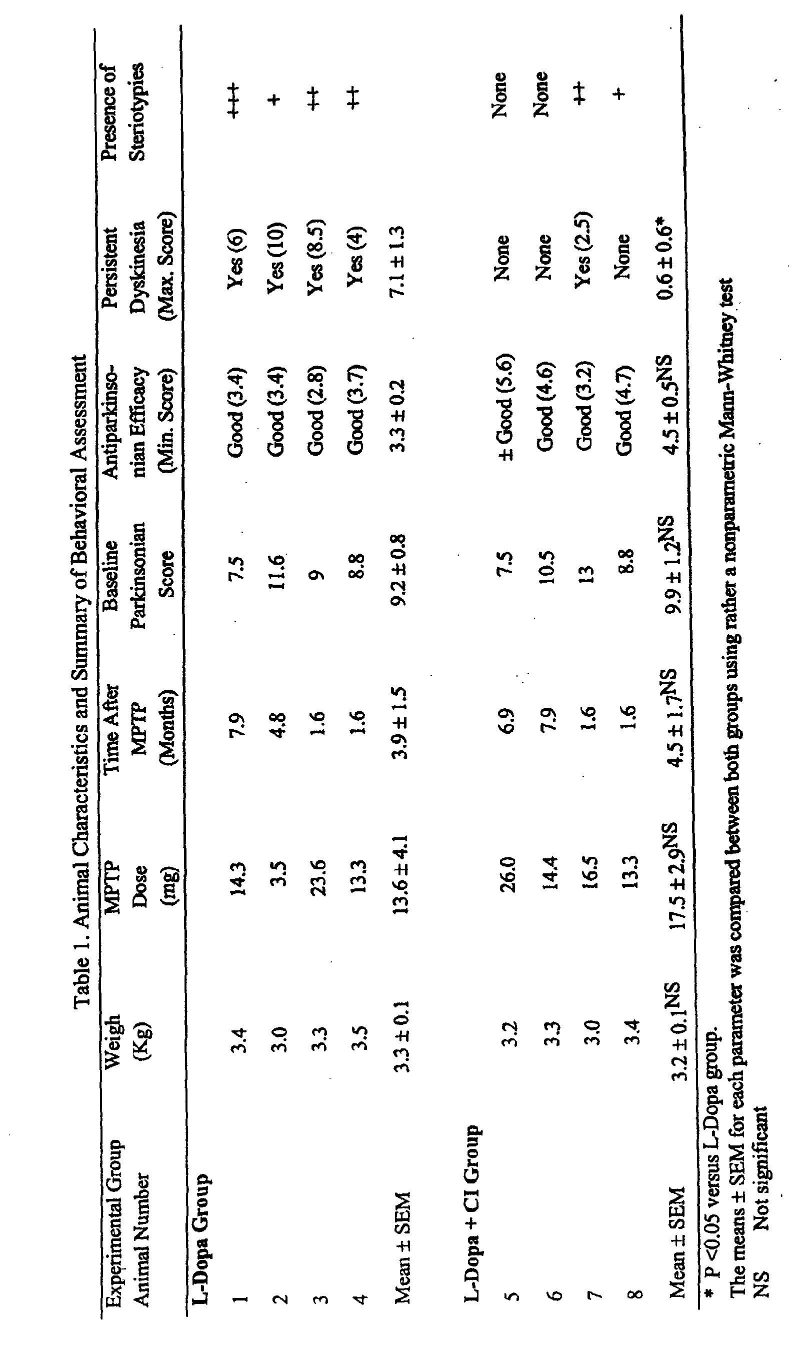

### Experimental Drugs Treatments

During this study, the animals were assigned to two treatment groups with four individuals in each group. Animals in each treatment group had a moderate to severe parkinsonism and were divided so that the different parameters which possibly could interfere with dyskinesias induction (ie, parkinsonian score at baseline, time allowed for recovery after MPTP administration, total MPTP dose, etc.) were similar for the two groups (see Table 1). The L-Dopa group was started on a chronic daily oral treatment with L-Dopa/benserazide alone (100/25 mg; Prolopa®) (Hoffmann-La Roche Limited, Mississauga, Ontario). The L-Dopa/CI-1041 group was given L-Dopa (same dosage) plus CI-1041 (at the dose of 10 mg/kg). CI-1041 was dissolved and administered per os. During the experiments, drugs were administered at 9:30 AM, and the spontaneous behavior of the animals was observed until the effect of a given drug was terminated. In both groups, the animals were treated for 28 consecutive days.

### Evaluation of the Response

The parkinsonian syndrome following MPTP exposure and the relief of parkinsonism following the administration of L-Dopa/benseraside alone or with CI-1041 were evaluated, using an abnormal disability scale for MPTP monkey (Hadj T.A., Gregoire L., Bangassoro E., Bedard P.J. Sustained cabergoline treatment reverses levodopa-induced dyskinesias in parkinsonian monkeys. *Clin. Neuropharmacol*., 2000;23:195-202), by two experienced blinded observers in the following way: (a) Posture: normal=0, Flexed intermittent=1, flexed constant=2, crouched=3; (b) Mobility: normal=0, mild reduction=1, moderate reduction=2, severe reduction=3; (c) Gait: normal=0, slow=1, very slow=2, very slow with freezing=3; (d) Tremor: absent=0, mild acting tremor=1, moderate acting tremor=2, resting tremor=3; (e) Climbing: present=0, absent=1; (f) Grooming: present=0, absent=1; (g) Vocalization: present=0, absent=1; (h) Social interaction: present=0, absent=1. A score was given every 30 minutes reflecting observations of the preceding half-hour. The maximal disability score is 16.

The severity of dyskinesias was also rated every 30 minutes for each body segment: the face, neck, trunk, arms, and legs in the following way: None=0; Mild=1 Moderate=2; Severe=3. The difference between mild, moderate, and severe dyskinesias for a given body segment is based on the assessment of the amplitude of the abnormal movements and the frequency (whether they are occasional, intermittent, or constant); each body segment being scored separately based on assessment of observation in the preceding half hour. The dyskinetic score obtained was the sum of the scores for all body segments for a maximal score of 21 points. Dyskinesias were mainly choreic in nature but dystonia was also seen. Stereotypies or licking were not considered as dyskinesias.

Locomotor activity of all monkeys was monitored using an electronic motility monitoring system fixed on each cage (Dataquest IV, Data Sciences Inc, St. Paul, MN). Using radio-waves frequency, a probe implanted subcutaneously in the back of each animal transmits the signal to a receiver attached to the cage which is connected to a computer. Counts of locomotor activity per 5 minutes for each animal were accumulated.

### Statistical Analysis

In each group, the total mobility counts recorded individually for 1 hour period during the peak effect after a given treatment were averaged for all monkeys and compared using an analysis of variance (ANOVA) for repeated measures followed by a Fisher PLSD test (least significant differences). The null hypothesis is rejected at the 0.05 level. The minimal parkinsonian score, as well as the maximal dyskinetic score obtained during an observed effect, were averaged for all monkeys in both L-Dopa or L-Dopa+CI groups. These averaged scores were compared using a nonparametric Mann-Whitney test, which was followed by a multiple comparisons test when the null hypothesis was rejected (post-hoc analysis).

### RESULTS

### Dyskinesia Induction

Figure 1 clearly shows that dyskinesias were significantly different in the two groups (P<0.018). Indeed, in the L-Dopa-treated group the animals rapidly developed dyskinesias and from the second week on, all animals had abnormal movements that gradually became moderate to severe at the end of the study (Figure 1). Dyskinesias were mainly choreic in nature but dystonic movements of the lower limbs were also seen. Conversely, co-administration of C-1041 completely prevented the induction of dyskinesias in three animals in which no dyskinesias was noted until the end of the study. In the L-Dopa+CI group, only one monkey developed mild choreic movements of the lower limbs at the end of the fourth week of treatment (see Figure 1).

### Antiparkinsonian Response

Figure 2A illustrates the decrease in the parkinsonian scores for each treatment group indicating a significant antiparkinsonian action. Both L-Dopa alone or with CI-1041 significantly (P<0.01) lowered the parkinsonisonian scores as compared to the control (see Figure 2A). Co-administration of CI-1041 with L-Dopa induced a similar effect as compared to L-Dopa given alone (see Figure 2A). However, the decrease in the parkinsonian score was slightly less important in L-Dopa+CI-treated group. The antiparkinsonian response was also reflected by the significant improvement in the locomotor activity in both groups which indicates effective reversal of parkinsonian akinesia. In fact, L-Dopa alone or with CI-1041 produced a significant (P<0.01) increase in locomotion as compared to control (see Figure 2B). The effect of the combined treatment regimen was not different (P<0.2) nor the total cumulative locomotor counts over the entire duration of the study which were comparable in both drug-treatment groups (data not shown). This significant stimulatory effect on locomotion was maintained during the 4 weeks (see Figure 2B). The delay and duration of the L-Dopa response was not significantly affected by CI-1041 co-administration (see Table 2). However, a trend for a delayed response was observed with CI-1041 mainly for the first 2 weeks of the treatment period.

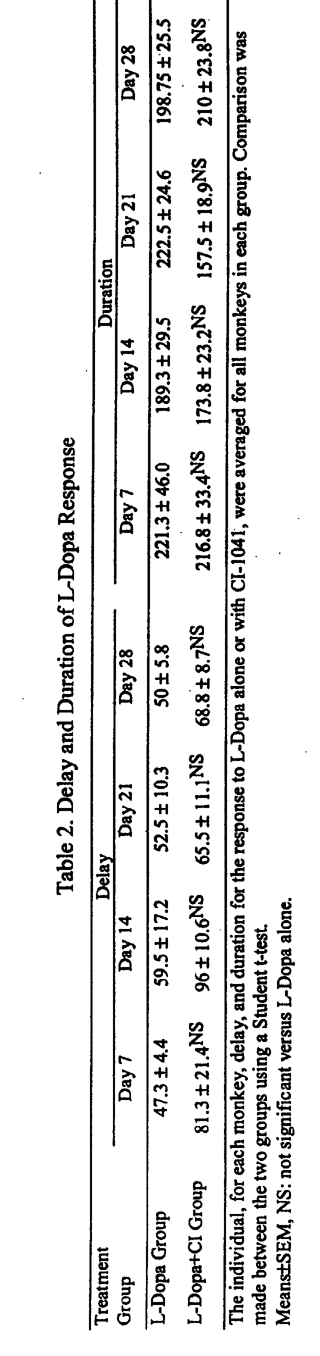

### Side Effects

CI-1041 was well-tolerated and no emesis, ataxia, or hallucinations were observed during the 1-month treatment.

Other variations and modifications of this invention will be obvious to those skilled in this art. This invention is not limited except as set forth in the following claims.

In the description the term "lower alkyls" means a straight or branched hydrocarbon radical having from one to six carbon atoms and includes, for example, methyl, ethyl, n-propyl, isopropyl, n-butyl, sec-butyl, isobutyl, tert-butyl, n-pentyl, n-hexyl, and the like.

The term "aryl" means an aromatic radical which is a phenyl group, a benzyl group, a naphthyl group, a biphenyl group, a pyrenyl group, an anthracenyl group, a fluarenyl group or a fused ring resulting from any two of phenyl, naphthyl, and a 5- or 6- membered ring containing from 0 to 3 heteroatoms selected from quinolones, isoquinolones, indoles, indanes, benzofurans, benzothiophenes, benzoxazoles, benzothiazoles, benzisoxazoles, coumarins, benzimidazoles and the like, unsubstituted or substituted by 1 to 3 substituents selected from alkyl, alkoxy, thioalkoxy, amino, formyl, carboxy, nitrile, -NHCOR, -CONHR, -CO₂R, -COR, or aryl.

"Halogen" is fluorine, chlorine, bromine or iodine.

## Claims

1. Use of NR1A/2B site-selective NMDA receptor antagonist compound for the manufacturing of pharmaceuticals for preventing dyskinesias.

2. The use of Claim 1, wherein the compound has the structure 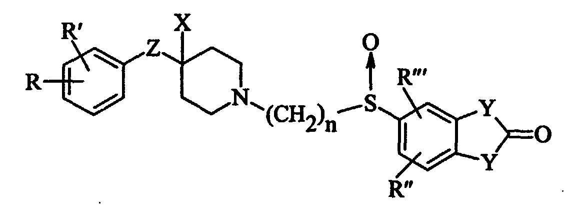 or stereoisomers or a pharmaceutically acceptable salt thereof, wherein R and R' are independently selected from the group consisting of hydrogen, hydroxy, alkyl, halogen, nitro, cyano, carboxaldehyde, aldehyde amine, C₁-C₆ alkoxy carbonylmethyl, hydroxy C₁-C₆ alkyl, aminocarbonylmethyl, hydrazinocarbonylmethyl, acetamido, aryl, aralkyl, amino, a halogenated alkyl group, a C₁-C₆ alkyl amino group, a C₁-C₆ alkyl amino group, or a C₁-C₆ alkoxy group;
R" and R''' are independently selected from the group consisting of hydrogen, hydroxy, alkyl, halogen, amino, a halogenated alkyl group, a C₁-C₆ alkyl amino group, or a C₁-C₆ alkoxy group;
X is hydrogen or hydroxy;
Z is -CH₂- or 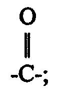
N is 2 to 4; and
Y is O, NH, or S.

3. The use of Claim 1, wherein the compound is selected from the group consisting of 6-{2-[4-(4-chloro-benzyl)-piperidin-1-yl]-ethanesulfinyl}-3H-benzooxazol-2-one; 6-{2-[4-(4-fluoro-benzyl)-piperidin-1-yl]-ethanesulfinyl}-3H-benzooxazol-2-one; 6-{2-[4-(4-methyl-benzyl)-piperidin-1-yl]-ethanesulfinyl}-3H-benzooxazol-2-one; 6-[2-(4-benzylpiperidin-1-yl)-ethanesulfinyl]-3H-benzooxazol-2-one; 6-{2-[4-(4-methoxybenzyl)-piperidin-1-yl]-ethanesulfinyl}-3H-benzooxazol-2-one; 6-{2-[4-(3,4-dichlorobenzyl)-piperidin-1-yl]-ethanesulfinyl}-3H-benzooxazol-2-one; 6-{2-[4-(2-fluorobenzyl)-piperidin-1-yl]-ethanesulfinyl}-3H-benzooxazol-2-one; 6-[2-(4-benzyl-4-hydroxypiperidin-1-yl)-ethanesulfinyl]-3H-benzooxazol-2-one; 6-{2-[4-(4-fluorobenzyl)-4-hydroxy-piperidin-1-yl]-ethanesulfinyl}-3H-benzooxazol-2-one; 6-[2-(4-benzoylpiperidin-1-yl)-ethanesulfinyl]-3H-benzooxazol-2-one; 6-{2-[4-(2,3-difluorobenzyl)-piperidin-1-yl]-ethanesulfinyl}-3H-benzooxazol-2-one; 6-{2-[4-(2,4-difluorobenzyl)-piperidin-1-yl]-ethanesulfinyl}-3H-benzooxazol-2-one; 6-{2-[4-(4-trifluoromethylbenzyl)-piperidin-1-yl}-3H-benzooxazol-2-one; 6{2-[4-(2,6-difluorobenzyl)-piperidin-1-yl]-ethanesulfinyl}-3H-benzooxazol-2-one; 6-{2-[4-(2,4-dichlorobenzyl)-piperidin-1-yl]-ethanesulfinyl}-3H-benzooxazol-2-one; N-(4-{1-[2-(2-oxo-2,3-dihydrobenzooxazol-6-sulfinyl)ethyl]piperidin-4-ylmethyl}phenyl)acetamide; 6-[2-(4-benzylpiperidin-1-yl)ethanesulfinyl]-5-chloro-3H-benzooxazol-2-one; 5-chloro-6-{2-[4-(4-fluorobenzyl)piperidin-1-yl]ethanesulfinyl}-3H-benzooxazol-2-one; (+)-6-{2-[4-(4-fluorobenzyl)-piperidine-1-yl]-ethanesulfinyl}-3H-benzooxazol-2-one; (-)-6-{2-[4,(4-fluorobenzyl)-piperidin-1-yl]ethanesulfinyl}-3H-benzooxazol-2-one and pharmaceutically acceptable salts thereof.

4. The use according to Claim 1, wherein the compound is 6-[2-[-(4-fluoro-benzyl)-piperidin-1-yl]-ethanesulfinyl]-3H-benzooxazol-2-one or a pharmaceutically acceptable salt thereof.

5. The use of Claim 1, wherein the compound has the structure 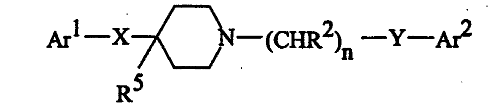 or a stereoisomer or pharmaceutically acceptable salt thereof wherein
Ar¹ and Ar² are independently aryl or a heteroaryl group, either of which may be independently substituted by one to three of hydroxy, alkyl, halogen, nitro, cyano, carboxaldehyde, aldehyde oxime, C₁-C₆ alkoxy carbonylmethyl, hydroxy C₁-C₆ alkyl, aminocarbonylmethyl, hydrazinocarbonylmethyl, acetamido, aryl, aralkyl, amino, a halogenated alkyl group, a C₁-C₆ alkyl amino group, or a C₁-C₆ alkoxy group;
X is -(CHR³)ₘ-, wherein each R³ is independently hydrogen, hydroxy or a lower alkyl group having 1 to 6 carbon atoms; and m is 0 or 1;
each R² is independently hydrogen, hydroxy, or a lower alkyl group having 1 to 6 carbon atoms;
n is 1,2,3 or 4;
Y is C≡C, O, and SOₚ wherein p is 0, 1, or 2, NR⁴ wherein R⁴ is hydrogen or a lower alkyl group having 1 to 6 carbon atoms, or a single bond; and
R⁵ is hydrogen or hydroxy.

6. The use of Claim 4, wherein the compound is selected from the group consisting of 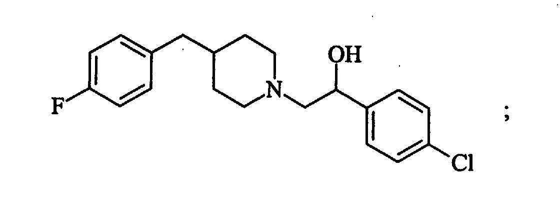 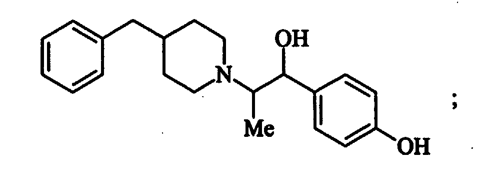 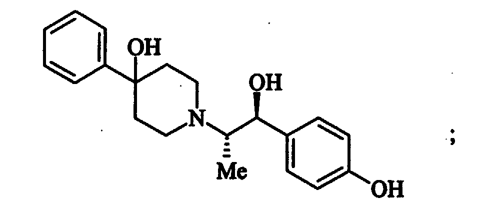 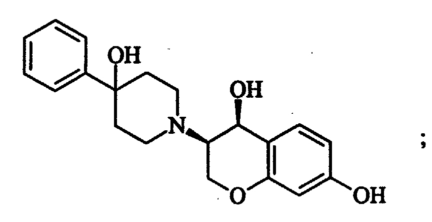 and 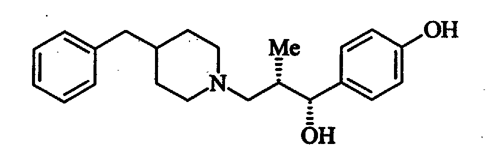 and their stereoisomers and pharmaceutically acceptable salts.

7. The use of Claim 4, wherein the compound is 1-[2-(4-hydroxyphenoxy)ethyl]-4-hydroxy-4-(4-methylbenzyl)piperidine hydrochloride.

8. The use of Claim 4, wherein the compound is 1-[2-(4-Hydroxyphenoxy)ethyl]-4-(4-fluorobenzyl)-4-hydroxy-piperidine hydrochloride.

9. The use of Claim 5, wherein the compound is [S-(R*,R*)]-4-hydroxy-α-(4-hydroxyphenyl)-β-methyl-4-phenyl-1-piperidineethanol.

10. Use of a NR1A/2B site-selective NMDA receptor antagonist compound for the manufacturing of pharmaceuticals for preventing dyskinesias in treatment of Parkinson's disease.

11. The use of Claim 10, wherein the NR1A/2B site-selective NMDA receptor antagonist is used in combination with a compound selected from L-Dopa, apomorphine, pramipexole, ropinirole, cabergoline, bromocryptine, pergolide, U-91356A, SKF 38393, SKF 82958, A-77636, A-86929, CY-208-243, any other selective or nonselective dopamine or other combined dopamine D1, D2, D3, D4, or D5 receptor agonists, or combinations thereof.
